# EUROPEAN PATENT SPECIFICATION

(11) **EP 2 968 092 B1**
(45) Date of publication and mention of the grant of the patent: **27.05.2020**
(21) Application number: 14714147.7
(22) Date of filing: 14.03.2014
(51) Int. Cl.: A61K 8/39, A61Q 1/08, A61Q 5/00, A61K 8/86, C08G 65/26, C08L 71/02

(54) **ALKOXYLATED FATTY ALCOHOL ALKYL ETHERS AND PRODUCTS CONTAINING SAME**
ALKOXYLIERTE FETTALKOHOLALKYLETHER UND PRODUKTE DAMIT
ETHERS ALKYLIQUES D'ALCOOLS GRAS ALCOXYLÉS ET PRODUITS LES CONTENANT

(30) Priority: 15.03.2013 US 201361787142 P
(43) Date of publication of application: 20.01.2016
(73) Proprietor: Croda, Inc., Edison, NJ 08837 (US)
(72) Inventor: PEREIRA, Abel G., Bridgewater, NJ 08807 (US); GUNDERMAN, Erik, Readington, NJ 08822 (US)
(74) Representative: Karfopoulos, Alexis Theo
(86) International application number: PCT/US2014/027644
(87) International publication number: WO 2014/143667

(56) References cited:
- EP-A1- 0 137 983
- EP-A1- 0 520 585
- EP-A2- 0 176 797
- EP-A2- 0 307 083
- EP-A2- 0 324 340
- EP-A2- 0 326 795
- WO-A1-93/20157
- WO-A1-96/36583
- WO-A1-03/022048
- CN-A- 1 464 001
- CN-A- 103 121 941
- DE-A1- 2 214 974
- DE-A1- 3 928 602
- DE-C1- 3 744 525
- JP-A- H1 161 644
- US-A- 4 753 885

## Description

### CROSS-REFERENCE TO RELATED APPLICATIONS

The present application claims the benefit of the filing date of U.S. Provisional Application No. 61/787,142, filed March 15, 2013, entitled Alkoxylated Fatty Alcohol Alkyl Ethers, Their Use and Products Containing Same.

### BACKGROUND OF THE INVENTION

The invention relates to alkoxylated fatty alcohol alkyl ethers and personal care products including the same. For example, personal care products can include anti-perspirants, moisturizing lotions, hair conditioners, shampoos, sun screens, and the like.

Alkoxylated fatty alcohols have been used in the art as emollients and/or solvents in personal care products. Alkoxylated fatty alcohols may include PPG-3 myristyl alcohol, or other alkoxylated fatty alcohols. However, products including certain alkoxylated alcohols used as emollients can have a sticky or tacky feel that can detract from a pleasant sensory perception of the product using them when applied. Further, products including alkoxylated alcohols can have limited spreadability due to unduly high viscosity.

Alkoxylated alcohol alkyl ethers used in various industries can be found, for example, in Japanese Patent No. 11-349983A(1999), Japanese Patent No. 2012-106959A, as well as in United States Patent Nos. 4,753,885 and 5,151,269.

There remains a need for improved materials for use in personal care products and for improved products made with them.

### BRIEF SUMMARY OF THE INVENTION

The present invention is directed to alkoxylated alcohol alkyl ethers. In some embodiments, these ethers include a very short chain ether end cap, such as a methyl or ethyl group. In some embodiments, the long chain fatty alcohol used is unsaturated and/or branched.

According to a first aspect of the present invention, there is provided a compound as defined in the claims.

In some embodiments, a compound of the invention includes:

R₁O-(PO)ₙ - R₂ (Formula Xa)

wherein P is a branched alkyl group having 3 carbons; n is an integer ranging from 2 to 10; R₁ is a branched or unsaturated hydrocarbon having 16 to 24 carbons; and wherein R₂ is a methyl group.

In some embodiments, n is 3. In some embodiments, R1 is an isostearyl group. In some embodiments, R1 is a behenyl group. In some embodiments, n is 3 and R₁ is an isostearyl group. In some embodiments, n is 3 and R₁ is a behenyl group. In some embodiments, n is 6. In some embodiments, n is 6 and R₁ is an isostearyl group.

In some embodiments, a compound of the invention includes:

R₁O-(PO)ₙ-R₂ (Formula Xa)

wherein P is a branched alkyl group having 3 carbons; n is an integer ranging from 2 to 10; R₁ is branched or unsaturated hydrocarbon having 14 to 24 carbons; and R₂ is ethyl group.

In some embodiments, n ranges from 3 to 4. In some embodiments, R₁ is unsaturated. In some embodiments, n is 4. In some embodiments, R₁ is a cetearyl group. In some embodiments, n is 4 and R₁ is a cetearyl group.

The present disclosure includes:

R₁O-(EO)ₘ-R₂ (Formula Xb)

wherein E is an alkyl group having 2 carbons; m is an integer ranging from 2 to 6; R₁ is branched or unsaturated hydrocarbon having 14 to 24 carbons which are substituted or unsubstituted; and R₂ is a methyl or ethyl group.

The present disclosure includes:

R₁O-(PO)ₙ-(EO)ₘ-R₂ (Formula Xc)

wherein P is a branched alkyl group having 3 carbons; E is an alkyl group having 2 carbons; n and m are integers, n ≥ 1, m ≥ 1, and n+m = 2 to 25; R₁ is branched or unsaturated hydrocarbon having 16 to 24 carbons, which are substituted or unsubstituted; R₂ is a methyl or ethyl group; and PO and EO can be in any order.

The present disclosure includes a compound of Formula X:

R₁O - A - R₂ (Formula X)

wherein:
i) A is one selected from the group consisting of:
   a) (PO)ₙ, where P is a branched alkyl group having 3 carbons and n is an integer ranging from 2 to 10;
   b) (EO)ₘ, where E is an alkyl group having 2 carbons and m is an integer ranging from 2 to 20; and
   c) (PO)ₙ-(EO)ₘ, where P is a branched alkyl group having 3 carbons, E is an alkyl group having 2 carbons, n and m are integers, n ≥ 1, m ≥ 1, and n+m = 2 to 25, and PO and EO can be in any order;
ii) R₁ is branched or unsaturated hydrocarbon having 8 to 24 carbons which are substituted or unsubstituted; and
iii) R₂ is a linear or branched alkyl group having 3 to 5 carbons, or a substituted or unsubstituted aromatic ring.

The present disclosure includes:

R₁O-(PO)ₙ-R₂ (Formula Xa)

wherein P is a branched alkyl group having 3 carbons; n is an integer ranging from 2 to 10; R1 is branched or unsaturated hydrocarbon having 8 to 24 carbons which are substituted or unsubstituted; and R2 is a linear or branched alkyl group having 3 to 5 carbons, or a substituted or unsubstituted aromatic ring.

In some embodiments, n is 3. In some embodiments, R₁ is an isostearyl group. In some embodiments, R₂ is a benzyl group. In some embodiments, n is 3, R₁ is an isostearyl group, and R₂ is a benzyl group.

The present disclosure includes:

R₁O-(EO)ₘ-R₂ (Formula Xb)

wherein E is an alkyl group having 2 carbons; m is an integer ranging from 2 to 20; R₁ is branched or unsaturated hydrocarbon having 8 to 24 carbons which are substituted or unsubstituted; and R₂ is a linear or branched alkyl group having 3 to 5 carbons, or a substituted or unsubstituted aromatic ring.

The present disclosure includes:

R₁O-(PO)ₙ-(EO)ₘ-R₂ (Formula Xc)

wherein P is a branched alkyl group having 3 carbons; E is an alkyl group having 2 carbons; n and m are integers, n ≥ 1, m ≥ 1, and n+m = 2 to 25; R₁ is branched or unsaturated hydrocarbon having 8 to 24 carbons which are substituted or unsubstituted; R₂ is a linear or branched alkyl group having 3 to 5 carbons, or a substituted or unsubstituted aromatic ring; and PO and EO can be in any order.

In some embodiments, n is 3. In some embodiments, m is 4. In some embodiments, n is 3 and m is 4. In some embodiments, R₁ is a behenyl group. In some embodiments, R₂ is a benzyl group. In some embodiments, n is 3, m is 4, R₁ is a behenyl group, and R₂ is a benzyl group.

The inventors have also discovered that by including some of the above described ethers into appropriate personal care products, these products can benefit from the improved properties of the compounds, thus providing more useful and desirable products. For example, by including certain emollients where the terminal hydroxyl group of certain alkoxylated fatty alcohols of the invention are capped by an alkyl group, such a methyl, ethyl, benzyl or the like, certain products can be produced having improved sensory feel in terms of stickyness and/or tackiness. Further, certain products can have improved spreadability due to reduced viscosity of some of the emollients of the invention.

According to a second aspect of the present invention, there is provided a personal care product as defined in the claims.

The present disclosure includes a personal care product including (I) at least about 0.25 % by weight of a compound of Formula Xa:

R₁O-(PO)ₙ- R₂ (Formula Xa)

wherein P is a branched alkyl group having 3 carbons; n is an integer ranging from 2 to 10; R₁ is a branched or unsaturated hydrocarbon having 8-24 carbons which are substituted or unsubstituted; and R₂ is a methyl group, an ethyl group, a linear or branched alkyl group having 3 to 5 carbons, or a substituted or unsubstituted aromatic ring; and (II) a dermatologically acceptable excipient.

In some embodiments, wherein R₂ is a methyl group. In some embodiments, R₁ is a branched or unsaturated hydrocarbon having 16 to 24 carbons which are substituted or unsubstituted. In some embodiments, n is 3 and R₁ is isostearyl. In some embodiments, n is 6 and R₁ is an isostearyl group. In some embodiments, n is 3 and R₁ is a behenyl group. In some embodiments, R₂ is an ethyl group. In some embodiments, R₁ is a branched or unsaturated hydrocarbon having 14-24 carbons, which are substituted or unsubstituted. In some embodiments, n is 4 and R₁ is a cetearyl group.

In some embodiments, the dermatologically acceptable excipient is selected from the group consisting of: absorbents, anti-acne agents, anti-irritants, antiperspirants, anticaking agents, antifoaming agents, antimicrobial agents, antioxidants, antidandruff agents, astringents, binders, buffers, biological additives, botanical extracts, buffering agents, bulking agents, chelating agents, chemical additives, coupling agents, conditioners, colorants, cosmetic astringents, cosmetic biocides, denaturants, drug astringents, detergents, dispersants, external analgesics, film formers, foaming agents, fragrance components, humectants, keratolytics, opacifying agents, pH adjusters, preservatives, propellants, proteins, retinoids, reducing agents, sequestrants, skin bleaching agents, skin-conditioning agents, skin soothing agents, skin healing agents, softeners, solubilizing agents, lubricants, penetrants, plasticizers, solvents and co-solvents, sunscreening additives, salts, surfactants, oils, anti-aging actives, anti-wrinkle actives and vitamins.

In some embodiments, the excipient is a conditioner, and wherein the personal care product further comprises a compound comprising a tertiary or quaternary nitrogen.

In some embodiments, the excipient is an antiperspirant, and wherein the personal care product further comprises a compound comprising a tertiary or quaternary nitrogen.

In some embodiments, the personal care product is selected from the group consisting of: a shampoo, a conditioner, a conditioning shampoo, a body wash, a hand wash, a hand sanitizer, a cleanser, a hair coloring, a hair relaxer, a cosmetic, a skin care product, an organic sunscreen, an inorganic sunscreen, a deodorant, an anti-perspirant, a depilatory, a skin bronzer, an acid cream, an moisturizing lotion, a styling gel, a hair color creme, a hair conditioning rinse, a lip stick, and a cosmetic remover.

In some embodiments, the personal care product is stable to hydrolysis at a pH ranging from about 1 to about 14.

In some embodiments, a method comprising applying the personal care product to the human body. In some embodiments, the method further comprises removing the applied personal care product.

In some embodiments, a method of imparting conditioning to hair comprising applying the personal care product to hair. In some embodiments, the method further comprises removing the applied personal care product. In some embodiments, the step of removing further comprises rinsing the hair with water.

In some embodiments, a method of preserving the degree of hair color in synthetically colored hair comprising applying the personal care product to hair.

In some embodiments, a method of imparting coloring to facial tissues comprising applying the personal care product to facial tissues.

In some embodiments, R₂ is a methyl, an ethyl, or a benzyl group. In some embodiments, the personal care product is a deodorant. In some embodiments, the personal care product is a sunscreen lotion or gel. In some embodiments, the personal care product is a styling gel. In some embodiments, the personal care product is an acid cream. In some embodiments, the personal care product is a moisturizing lotion. In some embodiments, the personal care product is a conditioning shampoo. In some embodiments, the personal care product is a hair conditioning rinse. In some embodiments, the personal care product is a hair color creme. In some embodiments, the personal care product is a lip stick. In some embodiments, the personal care product is a hand sanitizer.

The present disclosure includes a personal care product comprising (I) at least about 0.25% by weight of a compound of Formula Xb:

R₁O-(EO)ₘ-R₂ (Formula Xb)

wherein E is an alkyl group having 2 carbons; m is an integer ranging from 2-20; R₁ is branched or unsaturated hydrocarbon having 8 to 24 carbons which are substituted or unsubstituted; and R₂ is a methyl group, an ethyl group, a linear or branched alkyl group having 3 to 5 carbons, or a substituted or unsubstituted aromatic ring; and (II) a dermatologically acceptable excipient.

In some embodiments, R₁ is a branched or unsaturated hydrocarbon having 14 to 24 carbons which are substituted or unsubstituted and n is 2 to 6. In some embodiments, R₂ is a methyl, an ethyl, or a benzyl group.

In some embodiments, the dermatologically acceptable excipient is selected from the group consisting of: absorbents, anti-acne agents, anti-irritants, antiperspirants, anticaking agents, antifoaming agents, antimicrobial agents, antioxidants, antidandruff agents, astringents, binders, buffers, biological additives, botanical extracts, buffering agents, bulking agents, chelating agents, chemical additives, coupling agents, conditioners, colorants, cosmetic astringents, cosmetic biocides, denaturants, drug astringents, detergents, dispersants, external analgesics, film formers, foaming agents, fragrance components, humectants, keratolytics, opacifying agents, pH adjusters, preservatives, propellants, proteins, retinoids, reducing agents, sequestrants, skin bleaching agents, skin-conditioning agents, skin soothing agents, skin healing agents, softeners, solubilizing agents, lubricants, penetrants, plasticizers, solvents and co-solvents, sunscreening additives, salts, surfactants, oils, anti-aging actives, anti-wrinkle actives and vitamins.

In some embodiments, the excipient is a conditioner, and wherein the personal care product further comprises a compound comprising a tertiary or quaternary nitrogen.

In some embodiments, the excipient is an antiperspirant, and wherein the personal care product further comprises a compound comprising a tertiary or quaternary nitrogen.

In some embodiments, the personal care product is selected from the group consisting of: a shampoo, a conditioner, a conditioning shampoo, a body wash, a hand wash, a hand sanitizer, a cleanser, a hair coloring, a hair relaxer, a cosmetic, a skin care product, an organic sunscreen, an inorganic sunscreen, a deodorant, an anti-perspirant, an acid cream, an moisturizing lotion, a styling gel, a hair color creme, a hair conditioning rinse, a lip stick, a depilatory, a skin bronzer, and a cosmetic remover.

In some embodiments, the personal care product is stable to hydrolysis at a pH ranging from about 1 to about 14.

In some embodiments, a method comprises applying the personal care product to the human body. In some embodiments, the method further comprises removing the applied personal care product.

In some embodiments, a method of imparting conditioning to hair comprises applying the personal care product to hair. In some embodiments, the method further comprises removing the applied personal care product. In some embodiments, the step of removing further comprises rinsing the hair with water.

In some embodiments, a method of preserving the degree of hair color in synthetically colored hair comprising applying the personal care product to hair.

In some embodiments, a method of imparting coloring to facial tissues comprises applying the personal care product to facial tissues.

In some embodiments, the personal care product is a deodorant. In some embodiments, the personal care product is a sunscreen lotion or gel. In some embodiments, the personal care product is a styling gel. In some embodiments, the personal care product is an acid cream. In some embodiments, the personal care product is a moisturizing lotion. In some embodiments, the personal care product is a conditioning shampoo. In some embodiments, the personal care product is a hair conditioning rinse. In some embodiments, the personal care product is a hair color creme. In some embodiments, the personal care product is a lip stick. In some embodiments, the personal care product is a hand sanitizer.

The present disclosure includes a personal care product comprising (I) at least about 0.25 % by weight of a compound of Formula Xc:

R₁O-(PO)ₙ-(EO)ₘ-R₂ (Formula Xc)

wherein P is a branched alkyl group having 3 carbons; E is an alkyl group having 2 carbons; n and m are integers, n ≥ 1, m ≥ 1, and n+m = 2 to 25; R₁ is branched or unsaturated hydrocarbon having 8 to 24 carbons which are substituted or unsubstituted; R₂ is a methyl group, an ethyl group, a linear or branched alkyl group having 3 to 5 carbons, or a substituted or unsubstituted aromatic ring; and PO and EO can be in any order; and (II) a dermatologically acceptable excipient.

In some embodiments, R₂ is a methyl, or an ethyl, or a benzyl group. In some embodiments, R₁ is a behenyl group. In some embodiments, R₂ is a benzyl group. In some embodiments, n is 3, m is 4, R₁ is a behenyl group, and R₂ is a benzyl group.

In some embodiments, the dermatologically acceptable excipient is selected from the group consisting of: absorbents, anti-acne agents, anti-irritants, antiperspirants, anticaking agents, antifoaming agents, antimicrobial agents, antioxidants, antidandruff agents, astringents, binders, buffers, biological additives, botanical extracts, buffering agents, bulking agents, chelating agents, chemical additives, coupling agents, conditioners, colorants, cosmetic astringents, cosmetic biocides, denaturants, drug astringents, detergents, dispersants, external analgesics, film formers, foaming agents, fragrance components, humectants, keratolytics, opacifying agents, pH adjusters, preservatives, propellants, proteins, retinoids, reducing agents, sequestrants, skin bleaching agents, skin-conditioning agents, skin soothing agents, skin healing agents, softeners, solubilizing agents, lubricants, penetrants, plasticizers, solvents and co-solvents, sunscreening additives, salts, surfactants, oils, anti-aging actives, anti-wrinkle actives and vitamins.

In some embodiments, the excipient is a conditioner, and wherein the personal care product further comprises a compound comprising a tertiary or quaternary nitrogen.

In some embodiments, the excipient is an antiperspirant, and wherein the personal care product further comprises a compound comprising a tertiary or quaternary nitrogen.

In some embodiments, the personal care product is selected from the group consisting of: a shampoo, a conditioner, a conditioning shampoo, a body wash, a hand wash, a hand sanitizer, a cleanser, a hair coloring, a hair relaxer, a cosmetic, a skin care product, an organic sunscreen, an inorganic sunscreen, a deodorant, an anti-perspirant, an acid cream, an moisturizing lotion, a styling gel, a hair color creme, a hair conditioning rinse, a lip stick, a depilatory, a skin bronzer, and a cosmetic remover.

In some embodiments, the personal care product is stable to hydrolysis at a pH ranging from about 1 to about 14.

In some embodiments, a method comprising applying the personal care product to the human body. In some embodiments, the method further comprises removing the applied personal care product.

In some embodiments, a method of imparting conditioning to hair comprises applying the personal care product to hair. In some embodiments, the method further comprises removing the applied personal care product. In some embodiments, the step of removing further comprises rinsing the hair with water.

In some embodiments, a method of preserving the degree of hair color in synthetically colored hair comprising applying the personal care product to hair.

In some embodiments, a method of imparting coloring to facial tissues comprises applying a personal care product to facial tissues.

In some embodiments, the personal care product is a deodorant. In some embodiments, the personal care product is a sunscreen lotion or gel. In some embodiments, the personal care product is a styling gel. In some embodiments, the personal care product is an acid cream. In some embodiments, the personal care product is a moisturizing lotion. In some embodiments, the personal care product is a conditioning shampoo. In some embodiments, the personal care product is a hair conditioning rinse. In some embodiments, the personal care product is a hair color creme. In some embodiments, the personal care product is a lip stick. In some embodiments, the personal care product is a hand sanitizer.

Methods of using these compounds to produce personal care products and methods of using the resulting personal care products are also part of the invention.

### DETAILED DESCRIPTION

The present invention is directed to alkoxlyated fatty alcohol alkyl ethers, formulations, compositions, or personal care products including the same, as well as methods of their formulation and use.

All percentages and ratios used herein are by weight of the total composition and all measurements made are at about room temperature and normal pressure unless otherwise designated. "Room temperature" as defined herein means a temperature ranging between 22°C and 26°C. All temperatures are in degrees Celsius unless specified otherwise.

The present invention can "comprise" (open ended) or "consist essentially of" the components of the present invention as well as other ingredients or elements described herein. As used herein, "comprising" means the elements recited, or their equivalent in structure or function, plus any other element or elements which are not recited. The terms "having" and "including" are also to be construed as open ended unless the context suggests otherwise.

As used herein, "consisting essentially of" means that the invention may include ingredients in addition to those recited in the claim, but only if the additional ingredients do not materially alter the basic and novel characteristics of the claimed invention. Preferably, such additional ingredients will not be present at all or only in trace amounts. However, it may be possible to include up to about 10% by weight of materials that could materially alter the basic and novel characteristics of the invention as long as the utility of the compounds (as opposed to the degree of utility) is maintained.

All ranges recited herein include the endpoints, including those that recite a range "between" two values. Terms such as "about," "generally," "substantially," and the like are to be construed as modifying a term or value such that it is not an absolute, but does not read on the prior art. Such terms will be defined by the circumstances and the terms that they modify as those terms are understood by those of skill in the art. This includes, at very least, the degree of expected experimental error, technique error and instrument error for a given technique used to measure a value.

It should be further understood that a description in range format is merely for convenience and brevity and should not be construed as an inflexible limitation on the scope of the invention. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 2.3, 3, 4, 5, 5.7 and 6. This applies regardless of the breadth of the range.

As used herein, when it is said that a component, such as hydrocarbon, aromatic ring, or the like, can be "substituted", substitutions can include, for example, halogens, hydroxides, carbonyl, carboxylic, or amino groups, or substituents including such groups, in place of hydrogen.

Note that while the specification and claims may refer to a final product or personal care product such as, for example, a shampoo or conditioner or a sun screen as containing a certain reactant or a certain amount of, for example, a specific alkoxylated alcohol alkyl ether, it may be difficult to tell from the product that any particular recitation is satisfied. Such a recitation may be satisfied, however, if the materials used prior to final production, for example, meet that recitation. Indeed, as to any property or characteristic of a final product which cannot be ascertained from the final product directly, it is sufficient if that property resides in the components recited just prior to production steps used to make the personal care product.

The present disclosure includes an alkoxylated fatty alcohol alkyl ether defined by Formula Xa:

R₁O-(PO)ₙ-R₂ (Xa)

wherein P is a branched alkyl group having 3 carbons; n is an integer ranging from 2 to 10; R₁ is a branched or unsaturated hydrocarbon having 8 to 24 carbons, which may be substituted or unsubstituted; and R₂ is a methyl group, an ethyl group, a linear or branched alkyl group having 3 to 5 carbon atoms, or a substituted or unsubstituted aromatic ring.

In some embodiments, R₁ is a branched or unsaturated hydrocarbon having 16 to 24 carbon atoms. In other embodiments, R₁ is a branched or unsaturated hydrocarbon having 14 to 24 carbon atoms.

In some embodiments of Formula Xa, P is a branched alkyl group having 3 carbons; n is an integer ranging from 2 to 10; R₁ is a branched or unsaturated hydrocarbon having 16 to 24 carbon atoms which may be substituted or unsubstituted; and R₂ is a methyl group. In some embodiments, n is 3. In yet other embodiments, n is 6. In some embodiments, R₁ is an isostearyl group. In other embodiments, R₁ is a behenyl group. In some embodiments, n is 3 and R₁ is a branched or unsaturated hydrocarbon having 16 to 24 carbon atoms. In other embodiments, n is 3 and R₁ is an isostearyl group. In yet other embodiments, wherein n is 3 and R₁ is a behenyl group. In some embodiments, n is 6 and R₁ is a branched or unsaturated hydrocarbon having 16 to 24 carbon atoms. In one embodiment, n is 6 and R₁ is an isostearyl group.

In some embodiments of Formula Xa, P is a branched alkyl group having 3 carbons; n is an integer ranging from 2-10; and R₁ is branched or unsaturated hydrocarbon having 8 to 24 carbons which may be substituted or unsubstituted; and R₂ is an ethyl group. In some embodiments, R₁ is a branched or unsaturated hydrocarbon having 14 to 24 carbon atoms. In one embodiment, R₁ is a cetearyl group. In further embodiments, n is 3 to 4. In other embodiments, R₁ is unsaturated. In some embodiments, n is 4 and R₁ is a cetearyl group.

In some embodiments of Formula Xa, P is a branched alkyl group having 3 carbons; n is an integer ranging from 2-10; wherein R₁ is branched or unsaturated hydrocarbon having 8 to 24 carbons which may be substituted or unsubstituted; and wherein R₂ is (a) a linear or branched alkyl group having 3 to 5 carbons, or (b) a substituted or unsubstituted aromatic ring. When the aromatic ring is substituted, it may have up to 5 substituents, which may be the same or different. In other embodiments, R₂ is a linear or branched alkyl group having 3 carbon atoms. In other embodiments, R₂ is a linear or branched alkyl group having 4 carbon atoms. In yet other embodiments, R₂ is a linear or branched alkyl group having 5 carbon atoms. In yet further embodiments, R₂ is an aromatic ring. In yet further embodiments, R₂ is a substituted aromatic ring having one or two substituents. In one embodiment, R₂ is a benzyl group. In other embodiments, n is 3. In yet other embodiments, R₁ is an isostearyl group. In one embodiments, n is 3, R₁ is an isostearyl group, and R₂ is a benzyl group.

The present disclosure includes an alkoxylated fatty alcohol alkyl ether defined by Formula Xb:

R₁O-(EO)ₘ-R₂ (Xb)

wherein E is an alkyl group having 2 carbons; n is an integer ranging from 2 to 20; R₁ is a branched or unsaturated hydrocarbon having 8 to 24 carbons which may be substituted or unsubstituted; and R₂ is a methyl group, an ethyl group, a linear or branched alkyl group having 3 to 5 carbons, or a substituted or unsubstituted aromatic ring.

In some embodiments, n is an integer ranging from 2 to 10. In other embodiments, n is an integer ranging from 2 to 6.

In some embodiments, R₁ is a branched or unsaturated hydrocarbon having 14 to 24 carbon atoms which may be substituted or unsubstituted. In other embodiments, R₁ is a branched or unsaturated hydrocarbon having 16 to 24 carbon atoms which are substituted or unsubstituted. In yet other embodiments, R₁ is a branched or unsaturated hydrocarbon having 18 to 24 carbon atoms which are substituted or unsubstituted.

In some embodiments of Formula Xb, E is an alkyl group having 2 carbons; m is an integer ranging from 2 to 20; R₁ is branched or unsaturated hydrocarbon having 8 to 24 carbons which may be substituted or unsubstituted; and R₂ is (a) a linear or branched alkyl group having 3 to 5 carbons, or (b) a substituted or unsubstituted aromatic ring. When the aromatic ring is substituted, it may have up to 5 substituents, which may be the same or different. In some embodiments, R₂ is a linear or branched alkyl group having 3 carbon atoms. In other embodiments, R₂ is a linear or branched alkyl group having 4 carbon atoms. In yet other embodiments, R₂ is a linear or branched alkyl group having 5 carbon atoms. In yet further embodiments, R₂ is an aromatic ring. In yet further embodiments, R₂ is a substituted aromatic ring having one or two substituents.

In some embodiments of Formula (Xb), E is an alkyl group having 2 carbons; m is an integer ranging from 2 to 6; R₁ is branched or unsaturated hydrocarbon having 14 to 24 carbons which may be substituted or unsubstituted; and R₂ is a methyl group.

In some embodiments of Formula (Xb), E is an alkyl group having 2 carbons; m is an integer ranging from 2 to 6; R₁ is branched or unsaturated hydrocarbon having 14 to 24 carbons which may be substituted or unsubstituted; and R₂ is an ethyl group.

The present disclosure includes an alkoxylated fatty alcohol alkyl ether defined by Formula Xc:

R₁O-(PO)ₙ-(EO)ₘ-R₂ (Xc)

wherein E is an alkyl group having 2 carbons; P is branched alkyl group having 3 carbons; n and m are integers, where n≥1, m≥1, and the sum of n and m is 2 to 25; R₁ is a branched or unsaturated hydrocarbon having 8 to 24 carbons, which may be substituted or unsubstituted; and R₂ is a methyl group, an ethyl group, a linear or branched alkyl group having 3 to 5 carbons, or a substituted or unsubstituted aromatic ring. While depicted as PO then EO in Formula Xc above, it will be understood to those of skill in the art that PO and EO in Formula Xc can be in any order. For example, R₁O- can be connected to PO or EO, and -R₂ can be connected to PO or EO. For example, PO and EO can be arranged in blocks, in an alternating pattern, or randomly.

In other embodiments of Formula (Xc), R₁ is a branched or unsaturated hydrocarbon having 16 to 24 carbons which may be substituted or unsubstituted. In other embodiments, R₁ is a branched or unsaturated hydrocarbon having 18 to 24 carbons which may be substituted or unsubstituted. In yet other embodiments, R₁ is a branched or unsaturated hydrocarbon having 20 to 24 carbons which may be substituted or unsubstituted. In one embodiment, R₁ is a behenyl group.

In other embodiments of formula (Xc), n is 3. In some embodiments, m is 4. In yet other embodiments, n is 3 and m is 4.

In other embodiments of Formula (Xc), P is a branched alkyl group having 3 carbons; E is an alkyl group having 2 carbons; n and m are integers, n ≥ 1, m ≥ 1, and the sum of n and m is 2 to 25; R₁ is a branched or unsaturated hydrocarbon having 8 to 24 carbons which are substituted or unsubstituted; R₂ is (a) a linear or branched alkyl group having 3 to 5 carbons, or (b) a substituted or unsubstituted aromatic ring; and PO and EO are in any order. When the aromatic ring is substituted, it may have up to 5 substituents, which may be the same or different. In some embodiments, R₂ is a linear or branched alkyl group having 3 carbon atoms. In other embodiments, R₂ is a linear or branched alkyl group having 4 carbon atoms. In yet other embodiments, R₂ is a linear or branched alkyl group having 5 carbon atoms. In yet further embodiments, R₂ is an aromatic ring. In yet further embodiments, R₂ is a substituted aromatic ring having one or two substituents. In one embodiment, R₂ is a benzyl group. In yet another embodiment, n is 3, m is 4, R₁ is a behenyl group, and R₂ is a benzyl group.

In some embodiments of Formula (Xc), P is a branched alkyl group having 3 carbons; E is an alkyl group having 2 carbons; n and m are integers, n ≥ 1, m ≥ 1, and the sum of n and m is 2 to 25; R₁ is branched or unsaturated hydrocarbon having 16 to 24 carbons which may be substituted or unsubstituted; R₂ is a methyl group; and PO and EO may be in any order.

In some embodiments of Formula (Xc), P is a branched alkyl group having 3 carbons; E is an alkyl group having 2 carbons; n and m are integers, n ≥ 1, m ≥ 1, and the sum of n and m is 2 to 25; R₁ is branched or unsaturated hydrocarbon having 16 to 24 carbons which may be substituted or unsubstituted; R₂ is an ethyl group; and PO and EO may be in any order.

In other embodiments of Formula (Xc), when R₂ is a methyl group or ethyl group, R₁ is a branched and/or unsaturated hydrocarbon having 16 to 24 carbons. In yet other embodiments of Formula (Xc), when R₂ is a methyl or ethyl group, R₁ is a branched and/or unsaturated hydrocarbon having 18 to 24 carbons. In yet further embodiments of Formula (Xc), when R₂ is a methyl or ethyl group, R₁ is a branched and/or unsaturated hydrocarbon having 20 to 24 carbons.

The alkoxylated fatty alcohol alkyl ether of the invention may be characterized as, for example, being a "PPG 3" or may be characterized as containing 3 moles of propylene oxide. These two types of terms are used synonymously. It will be appreciated that this means that while roughly 3 moles of propylene oxide may have been added to the reaction mixture per mole of fatty alcohol and per mole of alkyl ether, not all of the resulting alkoxylated fatty alcohol alkyl esters will include exactly 3 molecules of ethylene oxide per fatty alcohol and per alkyl ether molecule. It is believed that while the predominant fraction will contain 3 alkoxy groups, other fractions will contain more or less than 3 molecules of alkylene oxide per alkyl ether molecule. In any event, however, a reference to a PPG 3 isostearyl alcohol methyl ether or a isostearyl alcohol methyl ether made with 3 moles of propylene oxide refers to the reaction product of those two materials in those amounts.

The alkoxylated fatty alcohol alkyl ethers as described herein, such as in formulas (X), (Xa), (Xb), and (Xc) have three components: one or more alkoxy groups (*e.g*., EO or PO), a fatty alcohol (*e.g*., R₁O-), and an end cap group (*e.g.*, -R₂).

The one or more alkoxy groups are selected from a group consisting of a branched alkyl group having 3 carbons or a linear alkyl group having 2 carbons. Exemplary reagents that may be utilized to form the one or more alkoxy groups can include propylene oxide or ethylene oxide.

It will also be appreciated that describing fatty alcohols in accordance with the invention can be complex, depending on a number of factors including their origin (such as the feedstocks used to produce the fatty alcohols and the methods used to produce these feedstock materials), the reaction chemistry used, and whether the fatty alcohols are branched, linear, saturated, unsaturated, substituted, or unsubstituted.

When something is referred to herein as being, containing, or being made from, for example, an isostearyl alcohol group, that means that the predominant fraction (most abundant compared to the amount of any other fatty alcohols in the feedstock) of the fatty alcohols used to produce the alkoxylated fatty alcohol alkyl ethers in accordance with the present invention are branched C18 fatty alcohols. It may also mean that the fatty component of the final ether in the predominant fraction of ethers is a branched C18 group, as the context suggests. However, there may be many other fatty alcohol groups of varying chain lengths present in the raw material or feedstock which may also be converted into alkoxylated fatty alcohol alkyl ethers along with the C18 species. The resulting mixture is still used in accordance herewith and would be identified as an isostearyl containing material or a C18 material.

Feedstocks can also be oils containing glycerides of fatty acids from which the fatty alcohols can be derived. Here too, the relative abundance would still apply. Accordingly, if reference is made to a range of chain lengths such as, for example, C16-C24, it means that at least the predominant fraction of the fatty alcohols used to produce the alkoxylated fatty alcohol alkyl ethers, or found in the ethers themselves as the context suggests, would fall within that range of chain lengths.

Table A shows published approximate weight percentages of some of the C20+ components in some of the common oils that can be used as a source of fatty alcohols used to produce some of the alkoxylated fatty alcohol alkyl ethers of the invention:

**TABLE A**

| Substance | C²⁰:0 | C²⁰:1 | C²⁰:4 | C²⁰:5 | C²²:0 | C²²:1 | C²²:5 | C²²:6 | C²⁴:0 |
|---|---|---|---|---|---|---|---|---|---|
| Cod liver oil | 8.8-14.6% | | | 2.6-9% | | 4.6-13.3% | 1-2% | 8.6-19% | |
| Herring oil | | 1.5-19.2% | | 4.6-10.2% | | 2.8-19.9% | 1-3.7% | 3.8-24.1% | |
| Menhaden oil | | 0.9-2.7% | 0.6-1.2% | 10.2-13.5% | | 0.7-1.7% | 1.1-2.3% | 3.3-14% | |
| Pilchard (Sardine) oil | | 3.2% | 1.6% | 16.9% | | 3.6% | 2.5% | 12.9% | |
| HEAR oil | | 0.8-13.5% | | | | 20.1-59.4% | | | 0.1-1.4% |
| Mustard Seed oil | | 7% | | | | 44.2% | | | |

Accordingly, behenyl alcohol can be derived from rapeseed oil, especially high erucic rapeseed oil (HEAR oil), which typically contains 46% of C22:1 alkyl (erucic), 1.5% of C22:0 alkyl (behenic), and 11% of C20:1 alkyl (gadoleic) by weight. In one embodiment, the HEAR oil can be hydrogenated to yield a composition containing about 48% C22:0 alkyl (behenic) which can further be distilled to yield any desired higher concentrations of C22:0 alkyl (behenic) acids which are then further converted to fatty alcohols. Other oils that can include fatty acid containing glycerides that can be converted into fatty alcohols include, without limitation, palm oil (predominant fractions are palmitic and/or oleic fatty acids), coconut oil (lauric (C12) fatty acids are the predominant fraction), corn oil, cottonseed oil, olive oil, peanut oil, sesame oil, palm kermal oil, safflower oil, sunflower oil, soybean oil and the like. Of course, any source of fatty alcohols falling within the scope of the invention are contemplated.

Exemplary fatty alcohols that may be used in accordance with the invention can include myristal alcohol, behenyl alcohol, iso-branched alcohols, such as isocetyl alcohol, isostearyl alcohol, 2-hexyl decanol, or Guerbet alcohols, e.g., beta alkylated dimer alcohols, based on a reaction developed by Marcel Guerbet.

When reference is made to a branched or unsaturated hydrocarbon in connection with the fatty alcohols used herein, it will be understood that these alcohols can be branched, unsaturated, or both. The fatty alcohols could have a single branch or unsaturation or multiples of one or both. Moreover, branching and unsaturation can exist in the fatty alcohol or can be introduced into the compounds after creation of the alkoxylated fatty alcohol alkyl ethers. Similarly, the fatty alcohols used can be unsubstituted, or substituted with, for example, halogens, hydroxides, carbonyl, carboxylic, or amino groups, or substituents including such groups. This may be done before or after the formation of the alkoxylated fatty alcohol alkyl ethers. This is true for all of the alkoxylated fatty alcohol alkyl ethers of formulas (X), (Xa), (Xb), and (Xc) discussed herein.

The end cap group, R2, of the alkoxylated fatty alcohol alkyl ethers of the invention is selected from a group consisting of a methyl group, an ethyl group, an isopropyl group, an n-propyl group, an n-butyl group, a t-butyl group, an linear or branched alkyl group having 8 carbons, an aromatic ring, or substituted versions thereof, the substituted groups including halogens, hydroxides, or amino groups. The end cap group can be formed from, for example, alkyl halides. Exemplary alkyl halides that can be used include methyl chloride, ethyl chloride, benzyl chloride, octyl bromide, or other alkyl halides.

Alkoxylated fatty alcohol alkyl ethers in accordance with the invention include PPG-3 isostearyl methyl ether, PPG-6 isostearyl methyl ether, PPG-3 octyl benzyl ether, PPG-6 myristyl ethyl ether, PPG-2, PEG-10 2-octyldodecanol methyl ether, PPG-3 2-hexyl decanol methyl ether, PPG-3 Behenyl Methyl Ether, PPG-3 isostearyl benzyl ether, PPG-3, PEG-4 isostearyl benzyl ether, PPG-4 cetearyl ethyl ether, or other alkoxylated fatty alcohol alkyl ethers described by formulas (X), (Xa), (Xb), and (Xc).

Some of the alkoxylated fatty alcohol alkyl ether in accordance with the invention may include one or more improved properties relative to alkoxylated fatty alcohols which have a terminal hydroxyl group instead of the end cap group, -R2. By "improved" it is meant that desirable sensory properties of a personal care product including an alkoxylated fatty alcohol alkyl ether are obtained. Exemplary sensory properties can include, for example, improved (reduced) sticky or tacky feel, improved spreadability, or combinations thereof. Where the improved property is reduced viscosity, the alkoxylated fatty alcohol alkyl ethers in accordance with the invention have reduced viscosity by at least about 10 to about 20 % relative to the corresponding alkoxylated fatty alcohol.

The alkoxylated fatty alcohol alkyl ethers may be formed by any suitable process. In one embodiment, an alkoxylated fatty alcohol alkyl ether may be formed by reacting a fatty alcohol with at least one of propylene oxide or ethylene oxide in the presence of an alkaline catalyst to form an alkoxylated fatty alcohol. The resulting alkoxylated fatty alcohol can be further reacted with an alkali metal to form a metal alkoxide. The metal alkoxide can be reacted with an alkyl halide, followed by neutralization with a suitable acid to form an alkoxylated fatty alcohol alkyl ether.

Exemplary alkaline catalysts that may be used include sodium hydroxide, potassium hydroxide, sodium methylate and the like.

Exemplary alkali metals that can be used include sodium hydroxide, potassium hydroxide, sodium methylate and the like.

The amounts of reagents used in the formation of alkoxylated fatty alcohol alkyl ethers can vary. Typical reactions involve reacting one mole of fatty alcohol with the desirable number of moles of alkylene oxide in the presence of a base catalyst. A detailed example of this reaction process is found in US Patent No. 7,091,243, the description of which is hereby incorporated herein by reference. For example, when forming a structure of formula Xa where n is 3, a molar ratio of fatty alcohol to propylene may range from about 1 to about 3 using between about 0.05 to about 0.5 alkaline catalyst. When n = 6, the molar ratio may range from about 1 mole fatty alcohol to about 6 moles EO and the amount of catalyst may range from about 0.01% by weight to about 0.5% by weight. The amount of alkoxylated fatty alcohol may range from about 0.8 moles to about 1.1 moles relative to about 0.8 moles to about 1.1 moles of a metal oxide. The amount of metal alkoxide can range from about 0.8 moles to about 1.1 moles relative to about 1 mole to about 1.5 moles of an alkyl halide.

In one embodiment, an alkoxylated fatty alcohol of formula Xa where R1 is isostearyl, R2 is methyl, and n is 3 can be made by the following exemplary process. One mole of isostearyl alcohol is charged into a stainless steel pressure vessel along with between about 0.03 to about 0.4% w/w of sodium hydroxide and heated to between about 100 to about 1200C under vacuum. Three moles of propylene oxide is charged slowly at between about 1250C to about 1400C as known in the art, and into the vessel. Once reaction is complete, one mole of sodium hydroxide is added and vacuum pulled at about 100 to about 1200C, followed by the slow addition of about 1.1 moles of methyl chloride to obtain the desired product.

In one embodiment, an alkoxylated fatty alcohol of Formula Xb, where R1 is myristyl, R2 is ethyl, and n is 5 can be made by a similar process as in the preceding paragraph, where the fatty alcohol is myristyl alcohol alkylene oxide is (5 Moles) ethylene oxide, the metal oxide is potassium hydroxide and the alkyl halide is ethyl chloride.

In one embodiment, an alkoxylated fatty alcohol of formula (Xc), where R1 is cetyl, R2 is benzyl, n is 10, and m is 5, can be made by a similar process as in the preceding paragraph, where the fatty alcohol is cetyl alcohol, the alkylene oxide is (about 10 moles) ethylene oxide and (about 5 Moles) propylene oxide, the metal oxide is sodium methoxide and the alkyl halide is benzyl chloride.

In other embodiments, the alkoxylated fatty alcohol of formula (X) may comprise any of the following:

PPG-5 behenyl methyl ether; PEG-8 behenyl benzyl ether; PPG-3 behenyl methyl ether; PPG-3, PEG-3 cetostearyl methyl ether; PPG-3 isostearyl methyl ether; PPG-3 isostearyl benzyl ether; PEG-5 isostearyl methyl ether; PEG-5 isostearyl benzyl ether; PPG-3, PEG-5 isostearyl methyl ether; PPG-3 myristyl benzyl ether; PEG-5 myristyl benzyl ether; PPG-3 oleyl methyl ether; PEG-3 oleyl methyl ether; PPG-3 oleyl benzyl ether; PPG-3 oleyl ethyl ether; PPG-3 stearyl methyl ether; PPG-8 stearyl methyl ether.

### Formulations

Personal care products including alkoxylated fatty alcohol alkyl ethers in accordance with the invention may include a shampoo, a conditioner, a conditioning shampoo, a body wash, a hand wash, a hand sanitizer, a cleanser, a hair coloring, a hair relaxer, a cosmetic, a skin care product, a organic sunscreen, an inorganic sunscreen, a deodorant, an antiperspirant, a depilatory, a skin bronzer, and a cosmetic remover, lipstick, foundation, hair mousse, styling gel, anti-aging creams and lotions, acne products, an acid cream, an moisturizing lotion, a styling gel, a hair color creme, a hair conditioning rinse or the like.

The personal care products of the present invention may be applied at least to the human body, such as to hair, the scalp, facial tissues and the skin. Facial tissues can include lips, cheeks, eye lids, chin, neck, or any suitable part of the head. The personal care products can be removed at some time after application. Removal can include removal using water or another dermatologically acceptable solvent, such as a cosmetic remover or the like. The personal care products can be applied to impart conditioning to hair, which can be treated or untreated. For example, treated hair can include synthetically colored hair, or other types of hair treatments. The personal care products can be applied to preserve the degree of coloring in synthetically treated hair.

The inventors have discovered that personal care products including one or more of the alkoxylated fatty alcohol alkyl ethers in accordance with the invention may have one or more improved properties. Without limitation, these improved properties include improved (reduced) sticky or tacky feel, improved spreadability, or combinations thereof.

The personal care products of the present invention are generally produced by known methods, using known ingredients in standard amounts. However, generally, these products may include an amount of at least one of the alkoxylated fatty alcohol alkyl ethers of the invention. The amount of alkoxylated fatty alcohol alkyl ether in a personal care product may vary depending on the use of the alkoxylated fatty alcohol alkyl ether therein and the type of personal care product. Generally, these products may include at least about 0.25% by weight of at least one of the alkoxylated fatty alcohol alkyl ether of the invention. In other embodiments, the amount can range from about 0.1% to about 90% by weight. When used as a emollient/vehicle] in roll on anti-perspirants, the amount of alkoxylated fatty alcohol alkyl ether may range from about 1% to about 20 % by weight. When used as a emollient/solvent in a make-up remover, the amount of alkoxylated fatty alcohol alkyl ether may range from about 2% to about 75 % by weight. When used as an emollient/moisturizer in a skin care lotion, the amount of alkoxylated fatty alcohol alkyl ether may range from about 1% to about 40%. When used as an emollient/dispersing aid in a inorganic sunscreen, the amount of alkoxylated fatty alcohol alkyl ether may range from about 3% to about 35%.

The personal care products include at least one dermatologically acceptable excipient, which may be any suitable excipient known for use in such products including, without limitation, those identified in the section titled "Additional Ingredients" herein. For example, the personal care products may include, in a particular embodiment, a compound comprising a tertiary or quaternary nitrogen. Exemplary personal care products which in a compound comprising a tertiary or quaternary nitrogen can include hair conditioners, cationic skin care lotions, conditioning shampoos, or the like. The personal care product may include, in a particular embodiment, one or more of alkoxylated phosphate esters, nonalkoxylated phosphate esters, or mixtures thereof, as discussed below. Exemplary personal care products which may include the aforementioned esters can include hair conditioners and shampoos.

### Additional Ingredients

The compositions of the invention may also include a wide range of "additional" ingredients used to make the personal care products. Some suitable miscellaneous "additional" ingredients commonly used in the cosmetic and personal care industry are described in The CTFA Cosmetic Ingredient Handbook, (2nd Ed., 1992), which is incorporated by reference herein. More specifically these personal care products and formulations of the present invention can include one or more additives such as absorbents, anti-acne agents, anti-irritants, antiperspirants, anticaking agents, antifoaming agents, antimicrobial agents, antioxidants, antidandruff agents, astringents, binders, buffers, biological additives, botanical extracts, buffering agents, bulking agents, chelating agents, chemical additives, coupling agents, conditioners, colorants, cosmetic astringents, cosmetic biocides, denaturants, drug astringents, detergents, dispersants, external analgesics, emulsion stabilizers, film formers, foaming agents, fragrance components, humectants, keratolytics, opacifying agents, pH adjusters, preservatives, propellants, proteins, retinoids, reducing agents, sequestrants, skin bleaching agents, skin-conditioning agents (humectants, miscellaneous, and occlusive), skin soothing agents, skin healing agents, softeners, solubilizing agents, lubricants, penetrants, plasticizers, solvents and co-solvents, sunscreening additives, salts, essential oils, viscosity builders, and vitamins. When present, these additives are provided in an amount which is consistent with the desired use and end product.

### Sunscreening Additives

Sunscreening additives may include octinoxate, octisalate, oxybenzone, or mixtures thereof. The above-listed suncreeening additives can block one or more wavelengths of ultraviolet light. Sunscreen additives may include UV absorbing compounds such as CHROMAVEIL® which is available from Croda, Inc. and/or include a quaternary nitrogen compound, such as Quaternium-95, which acts as a UV filter for hair care applications.

If sunscreening additives are present in a final product composition, the amount may vary from about 0.2% to about 75%, preferably from about 2% to about 50% by weight of the composition.

### Preservatives

Examples of suitable preservatives include sodium methyl paraben, ethyl paraben and mixtures thereof. If preservatives are present in a final product composition, the amount may vary from about 0.01% to about 0.5%, preferably, from about 0.1% to about 0.2% by weight of the composition.

### Solubilizing agents

Exemplary alkoxylated fatty alcohols include Brij O100 or Brij O20 available from Croda, Inc., which include oleth-10 and oleth-20, respectively, or INCROCAS™ 30 which includes PEG-30 castor oil. If solubilizing agents are present in a final product composition, the amount may vary from about 0.2% to about 20%, preferably, from about 1% to about 10% by weight of the composition.

### Emulsion Stabilizers

Examples of suitable emulsion stabilizers include fatty alcohols, such as CRODACOL™ C70, available from Croda, Inc., which includes cetyl alcohol, or CRODACOL™ S70, which includes stearyl alcohol, as well as gums such a guar, carbomers and cellulose as non limiting examples. If emulsion stabilizers are present in a final product composition, the amount may vary from about 0.1% to about 75%, preferably, from about 0.2% to about 50% by weight of the composition.

### pH Adjusters

Any suitable base can be used to adjust the pH as needed. Amount needed will vary depending on the type of other ingredients used, type of system made and the desired pH. For example a hair relaxer will require a high level while a neutralizing shampoo will not. Examples of suitable pH adjusters include sodium hydroxide (NaOH), triethanolamine (TEA), and aminomethylpropanol, and mixtures thereof. However, citric, Lactic, phosphoric and other acids can also be used..

### Film Formers

Examples of suitable film formers include glycerin/diethylene glycol adipate copolymer, glycerin/diethylene glycol adipate/myristate copolymer, ethyl ester of PVM/MAcopolymer, PVP/dimethiconylacrylate/polycarbamyl/polyglycol ester, and mixtures thereof. If the film formers are present in the final product compositions, the amount may vary from about - 0.1% to about 20% by weight of the composition, preferably, from about 0.2% to about 10% by weight of the composition.

### Vitamins

Examples of suitable vitamins include ascorbic acid, tocopherol, tocopherol acetate, retinoic acid, retinol, and retinoids.

### Conditioning Agents

The personal care products of the present invention may be conditioners and or conditioning shampoos body washes, cleansers, hair colors and/or hair relaxers which may include hydrolyzed animal protein as additional conditioning agents. Croda Incorporated sells an example of a commercially available material under the trade name Crotein Q®. Other examples include INCROQUAT™ Behenyl TMS-50 which includes Behentrimonium methosulfate, cetyl alcohol, and butyl alcohol; or INCROQUAT™ Behenyl 18-MEA, which includes Behentrimonium methosulfate, cetyl alcohol, and Quaternium-33, stearamidopropyl PG-dimonium chloride phosphate, stearamidopropyl ethyldimonium ethosulfate, stearamidopropyl dimethyl (myristyl acetate) ammonium chloride, stearamidopropyl dimethyl cetearyl ammonium tosylate, stearamidopropyl dimethyl ammonium chloride, stearamidopropyl dimethyl ammonium lactate, or other examples including those described in U.S. Patent No. 6,607,715 and 6,638,497. Other examples include urea, glycerol, and propoxylated glycerols, including those described in U.S. Patent No. 4,976,953, which is incorporated by reference herein.

### Surfactants

In addition to the compositions of the invention, and particularly when used in connection with shampoos, surfactants, and in particular, surfactants that will not strip color, may be present in the compositions of the invention. These may include, without limitation, one or more nonionic surfactants, anionic surfactants, cationic surfactants, amphoteric surfactants, zwitterionic surfactants, and mixtures thereof. For some of surfactants that may be used in combination with the compositions of the invention, please see McCutcheon's, Detergents and Emulsifiers, (1986), U.S. Pat. Nos. 5,151,210, 5,151,209, 5,120,532, 5,011,681, 4,788,006, 4,741,855, U.S. Pat. No. 4,704,272, 4,557,853, 4,421,769, 3,755,560; all incorporated herein by reference in their entirety.

### Emulsifiers

The compositions of the invention may also include various emulsifiers. In the final product compositions of the invention, emulsifiers may be included in the amount of up to about 40% , preferably, in the amount of from about 2% to about 30% by weight of the composition. The examples of suitable emulsifiers include ethoxylated alcohols or sorbitan esters such as the Brij and Tween series respectively from Croda Inc. In some cases, cationinc emulsifiyers may be used such as INCROQUAT™ Behenyl TMS-50 which includes Behentrimonium methosulfate, cetyl alcohol, and butyl alcohol or similar cationic surfactants.

Emulsifers can include alkoxylated phosphate esters, nonalkoxylated phosphate esters or mixtures thereof. Mixtures of alkoxylated and nonalkoxylated phosphate esters which are useful are described and claimed in U.S. Patent No. 6,117,915 (hereinafter the '915 patent). Exemplary mixtures of nonalkoxylated and alkoxylated phosphate esters that may be used in accordance with the present invention include CRODAFOS CES, available from Croda, Inc., which is a mixture of PEG 10 cetyl alcohol phosphate esters and phosphate esters of cetyl alcohol with cetearyl alcohol as a carrier; CRODAFOS HCE, which is a PEG 5 oleyl phosphate ester and a nonalkoxylated di-oleyl phosphate ester. Also useful is CRODAFOS CS20 ACID, which consists of ceteth 20 phosphate and dicetylphosphate in cetearyl alcohol as a carrier.

### Thickeners

The compositions of the invention may also include various thickeners, such as cross-linked acrylates, nonionic polyacrylamides, xanthan gum, guar gum, gellan gum, and the like; polyalkyl siloxanes, polyaryl siloxanes, and aminosilicones. In the final product compositions of the invention, thickeners may be included in the amount of up to about 8% on an actives basis, preferably, in the amount of from about 0.1% to about 4% on an active basis by weight of the composition. The specific examples of the suitable thickening silicon compounds include polydimethylsiloxane, phenylsilicone, polydiethylsiloxane, and polymethylphenylsiloxane. Some of the suitable silicon compounds are described in European Patent Application EP 95,238 and U.S. Patent No. 4,185,017, which are incorporated herein by reference. The compositions of the invention may also include silicone polymer materials, which provide both style retention and conditioning benefits to the hair. Such materials are described in U.S. Patent No. 4,902,499, which is incorporated herein by reference.

### Colorants

Hair color examples can be found in patents such as U.S. Patent No. 4,865,618 (Junino et al.), which is incorporated herein by reference. Without setting any limitations, as an example, the invention herein can be incorporated into any of the application examples disclosed by Junino et al. starting in column 22. Simply one skilled in the art can just add, for example, about 3% w/w Crodafos HCE and 0.5% w/w Optasence CP-6 (Polyquaternium 6) to any of the application examples described by Junino et al. As is known to those familiar with the art, hair color (tint) formulations contain various dyes, couplers etc. as also described in Junino et al. in U.S. Patent No. 4,865,618, which is hereby incorporated by reference.

### Examples

As shown in Tables 1-9 below, weights are collective for all Parts. For example, as shown in Table 1, the sum of the weights in Parts A-C is 100%.

### Example 1: Sprayable Sunscreen Lotion

**Table 1**

| **Ingredients** | **Weight %** |
|---|---|
| **Part A** | |
| Deionized Water | 61.20 |
| Xanthan Gum | 0.20 |
| CRODAFOS CS-20 ACID (Cetearyl Alcohol, Ceteth-20 Phosphate, Dicetyl Phosphate) | 4.00 |
| Glycerin | 5.00 |
| Triethanolamine (TEA, 98%) | 0.10 |

| **PART B** | |
|---|---|
| CRODAMOL AB (C12-C15 Alkyl Benzoate) | 3.00 |
| PPG-3 Isostearyl Methyl Ether | 5.00 |
| Octinoate | 7.50 |
| Octisalate | 5.00 |
| Oxybenzone | 5.00 |
| CHROMAVEIL (Cas# 1030827-59-8) | 3.00 |

| **PART C** | |
|---|---|
| Propylene Glycol, Diazolidinyl Urea, Methyl Paraben, Propyl Paraben | 1.00 |

The sprayable sunscreen disclosed in Table 1 may be formed by heating deionized water to a temperature between about 75 °C to about 80°C, then adding the xanthan gum, and allowing the xanthan gum to completely hydrate. The remaining ingredients of Part A in Table 1 may be added one at a time, each ingredient being allowed to fully dissolve before adding the next ingredient. Separately, Part B may be prepared by combining the ingredients of Part B in Table 1 and heating to about 75°C. Once Part A and Part B are prepared, Parts A and B can be combined and maintained at a temperature between about 75 °C to about 80°C for about 15 minutes. The mixture of Parts A and B is then cooled to about 45°C. Once cooled, Part C is added to the mixture of Parts A and B. The combined mixture may then be cooled and packaged.

### Example 2: Beta Hydroxy Acid Cream

**Table 2**

| **Ingredients** | **Weight %** |
|---|---|
| **Part A** | |
| CRODAFOS CS-20 ACID (Cetearyl Alcohol, Ceteth-20 Phosphate, Dicetyl Phosphate) | 8.0 |
| CRODACOL C-70 (Cetyl Alcohol) | 2.0 |
| Mineral Oil | 14.50 |
| CRODAMOL STS (PPG-3 Benzyl Myristate) | 3.00 |
| SUPER STEROL ESTER (C10-C30 Cholesterol/Lanosterol Esters) | 5.00 |
| Ethylhexylmethoxy Cinnanmate | 5.00 |

| **PART B** | |
|---|---|
| Salicylic Acid | 2.00 |
| PPG-3 Isostearyl Methyl Ether | 3.00 |

| **PART C** | |
|---|---|
| Deionized water | 52.20 |
| Glycerin | 5.00 |
| Sodium Hydroxide (pellets, 97%+) | 0.30 |

The acid cream disclosed in Table 2 may be formed by combining and heating the components of Part A to about 80°C. Part B is added to Part A, and the acid crystals are allowed to dissolve. Separately, the components of Part C may be combined and heated to about 80°C. Then, Part C may be added to the existing mixture of Parts A and B while mixing. The combined mixture of Parts A, B and C may be maintained at about 80°C for about 20 minutes, and then cooled to room temperature without using a water bath.

### Example 3: Microemulsion Styling Gel

**Table 3**

| **Ingredients** | **Weight %** |
|---|---|
| **Part A** | |
| INCROCAS 30 (PEG-30 Castor Oil) | 10.00 |
| Mineral Oil | 10.00 |
| Brij O10 (Oleth-10) | 10.00 |
| Brij O20 (Oleth-20) | 11.00 |
| INCROQUAT BEHENYL 18-MEA (Behentrimonium Methosulfate, Quaternium-33, Cetearyl Alcohol) | 2.00 |
| Propyl Paraben | 0.20 |

| **PART B** | |
|---|---|
| Deionized water | 40.60 |
| Glycerin | 7.00 |
| Polypropylene Glycol | 5.00 |
| PPG-3 Behenyl Methyl Ether | 4.00 |
| Methyl Paraben | 0.20 |

The styling gel disclosed in Table 3 may be formed by combining the ingredients of Part A while mixing and heating at a temperature between about 90°C to about 95°C. Separately, the ingredients of Part B can be combined while mixing and heating at a temperature between about 90°C to about 95°C. Then, Part B is added to Part A while rapidly mixing and maintaining the temperature for about 15 minutes. Then, the mixture of Parts A and B can be cooled to about 60°C and pour into molds before the mixture reaches a set point of about 50 to about 55°C, pH: 6.00 ±0.50.

### Example 4: Cationic Skin Moisturizing Lotion

**Table 4**

| **Ingredients** | **Weight %** |
|---|---|
| **Part A** | |
| Deionized water | 76 .98 |
| PPG-3 Behenyl Methyl Ether | 10.00 |
| Glycerin | 5.00 |
| CRODACOL S-70 (Stearyl Alcohol) | 2.52 |
| INCROQUAT BEHENYL TMS-50 (Behentrimonium Methosulfate, Butylene Glycol, Cetearyl Alcohol) | 2.50 |

| **PART B** | |
|---|---|
| Cyclomethicone | 2.00 |
| Polypropylene Glycol, Diazolidinyl Urea, Methyl Paraben, Propyl Paraben | 1.00 |

The moisturizing lotion disclosed in Table 4 may be formed by combining the ingredients of Part A while mixing and heating at a temperature between about 75°C to about 80°C. The heating temperature of Part A is maintained for about 15 minutes, and then the mixture of Part A is cooled to about 50°C. The ingredients of Part B are then be added individually to Part A while mixing. The lotion may then be cooled and packaged.

### Example 5: Conditioning Shampoo with UV Protection

**Table 5**

| **Ingredients** | **Weight %** |
|---|---|
| **Part A** | |
| Deionized Water | 29.22 |
| Glycerin | 1.00 |
| Disodium EDTA | 0.20 |
| PEG 7M | 0.20 |

| **PART B** | |
|---|---|
| Ammonium Lauryl Sulfate (ALS), 28% | 14.00 |
| Ammonium Lauryl Ether Sulfate (ALES), 25% | 40.00 |
| INCRONAM 30 (Cocamidopropyl Betaine) | 6.60 |
| LUSTREPLEX (Polyquaterium-70 (and) Dipropylene Glycol) | 1.42 |
| Glycol Distearate | 0.80 |
| CHROMAVEIL **(**cas# 1030827-59-8) | 2.50 |
| Silicone | 0.50 |
| PPG-3 Behenyl Methyl Ether | 2.00 |

| **PART C** | |
|---|---|
| CRODASONE W (Hydrolyzed Wheat Protein PG-Propyl Silanetriol) | 1.00 |
| CROSILK LIQUID (Silk Amino Acids) | 0.20 |
| Methylisothiazolinone | 0.10 |

| **PART D** | |
|---|---|
| CROTHIX LIQUID (PEG-150 Pentaerythrityl Tetrastearate (and) PEG-6 Caprylic/Capric Glycerides (and) Water) | 0.26 |

The conditioning shampoo disclosed in Table 5 may be formed by combining the initial three ingredients of Part A and mixing until the solid has dissolved. Then, the remaining ingredient, PEG 7M is added and mixed until hydrated. Then, the ingredients of Part B are added to the mixture of Part A at temperatures up to about 75 °C and mixed until solids have dissolved. The Part A/Part B mixture is cooled to about 40°C, and then the ingredients of Part C are added. Once the ingredients of Part C have been dissolved in the Part A/Part B mixture, the pH of the combined Part A/Part B/Part C mixture may be determined. If the pH is outside a range of about 5.5 to about 7.0, the pH is adjusted to be within that range. Then, the ingredients of Part D are added while slowly mixing between about 100 to about 300 rotations per minute (rpm). In some embodiments, a conditioning shampoo, for example, such as that of Example 5, may be applied to hair to at least one of impart conditioning to hair or maintain a degree of hair color in synthetically colored hair.

### Example 6: Hair Conditioning Rinse with UV Protection

**Table 6**

| **Ingredients** | **Weight %** |
|---|---|
| **Part A** | |
| Crodazozosoft DBQ (Quaternium 91 (and) Cetrimonium Methosulfate (and) Cetearyl Alcohol | 2.50 |
| Dimethicone | 5.00 |
| CRODACOL S-70 (Stearyl Alcohol) | 2.50 |
| Wheat Germ Oil | 1.00 |
| BHT | 0.10 |
| CHROMAVEIL (cas# 1030827-59-8) | 2.00 |
| PPG-3 Isostearyl Benzyl Ether | 2.00 |

| **PART B** | |
|---|---|
| Deionized Water | 80.90 |

| **PART C** | |
|---|---|
| Propylene Glycol (and) Diazolidinyl Urea (and) Methylparaben (and) Propylparaben | 1.00 |

| **PART D** | |
|---|---|
| HYDROTRITICUM WAA (Wheat Amino Acids) | 3.00 |

The hair conditioning rinse disclosed in Table 6 may be formed by combining the ingredients of Part A while mixing and heating at a temperature between about 80°C to about 85°C. Separately, the ingredients of Part B is heated at a temperature between about 80°C to about 85°C. Then, Part B is added to Part A while mixing and maintaining the temperature for about 15 minutes. Then, the mixture of Parts A and B is cooled to about 50°C. After cooling, the ingredients of Part C are added while mixing, followed by the ingredients of Part D. In some embodiments, a hair conditioning rinse, for example, such as that of Example 6, may be applied to hair to at least one of impart conditioning to hair or maintain a degree of hair color in synthetically colored hair.

### Example 7: Anti-Perspirant

**Table 7**

| **Ingredients** | **Weight %** |
|---|---|
| **Part A** | |
| PPG-6 Isostearyl Methyl Ether | 47.00 |
| ARLAMOL™ PM3 (PPG-3 Myristyl Ether) | 3.00 |
| ARLAMOL PB14 (PPG-14 Butyl Ether) | 2.00 |
| ARLAMOL PC10 (PPG-10 Cetyl Ether) | 2.00 |
| BRIJ™ S10 (Steareth-10) | 1.00 |

| **PART B** | |
|---|---|
| CRODACOL™ S95 (Stearyl Alcohol) | 16 .00 |
| Hydrogenated Castor Oil | 3.50 |
| Corn Starch Modified | 3.00 |
| Fumed Silica | 0.50 |

| **PART C** | |
|---|---|
| Aluminum Zirconium Tetrachlorohydrex GLY | 22.00 |

The anti-perspirant disclosed in Table 7 may be formed by combining the ingredients of Part A while mixing and heating at a temperature are then added to the mixture. Once the first two ingredients of Part A are dissolved, then the remaining ingredients of Part B are added. Each remaining ingredient of Part B is added individually. The mixture of Parts A and B is mixed until it is homogeneous. Then, the mixture is cooled to a temperature between about 60°C to about 65°C. While maintaining that temperature, the ingredients of Part C are added while mixing and the mixture is mixed until it is homogeneous. Then, the mixture is cooled to a temperature of about 50°C, and poured into molds to form a solid anti-perspirant.

### Example 8: Hair Color Creme

**Table 8**

| **Ingredients** | **Weight %** |
|---|---|
| **Part A** | |
| Deionized Water | 58.65 |
| Sodium Sulfite | 0.30 |
| EDTA | 0.20 |
| Sodium Isoascorbate | 0.30 |
| INCROMIDE™ CDEA (Cocamide DEA) | 3.00 |
| Glycerin | 0.50 |
| INCROMECTANT™ AMEA 100 (Acetamide MEA) | 0.50 |
| p-Phenylenediamine | 0.05 |
| 4-amino-2-hydroxytoluene | 0.80 |
| Resorcinol | 0.20 |
| 1-Napthol | 0.20 |
| p-aminophenol | 0.80 |

| **PART B** | |
|---|---|
| KERATINT EZ (Cetyl Alcohol (and) Stearyl Alcohol (and) PPG-5 Ceteth-20 & Dicetyl Phosphate (and) Ceteth-10 Phosphate (and) Behentrimonium Methosulfate) | 19.00 |
| PPG-3, PEG-4 Behenyl Benzyl Ether | 2.00 |

| **PART C** | |
|---|---|
| CRODASONE W (Hydrolyzed Wheat Protein PG-Propyl Silanetriol) | 2.00 |
| CROSILKQUAT (Cocamidopropyl Hydroxypropyl Silk Amino Acids) | 0.50 |
| CRODATERIC™ CAS 50 (Cocamidopropyl Hydroxysultaine) | 1.00 |

| **PART D** | |
|---|---|
| Ammonia | 10.00 |

The hair color creme disclosed in Table 8 may be formed by combining the ingredients of Part A while mixing and heating to a temperature of up to about 70°C, and until the ingredients are dissolved. Separately, the ingredients of Part B can be heated up to about 70°C. Then, Part B can be added to Part A while mixing and maintaining the temperature for about 15 minutes. Then, the mixture of Parts A and B are cooled to at temperature of about 45°C. After cooling, the ingredients of Part C are added while mixing. After further cooling or at a temperature of about 35°C, the ingredients of Part D are added to Parts A/B/C and the mixture be mixed for about 1 hour. The mixture is then mixed with a hydrogen peroxide developer at a weight ratio of about 1:1, at the time of application to hair.

### Example 9: Lip Stick

**Table 9**

| **Ingredients** | **Weight %** |
|---|---|
| **Part A** | |
| Syncrowax OSW (Tribehenin (and) Sorbitol/Sebacic Acid Coploymer Behenate) | 3.50 |
| Castor Oil | 23.55 |
| Crodamol PTIS (Pentaerythrityl Tetraisostearate) | 16.50 |
| PPG-4 Cetearyl Ethyl Ether | 11.00 |
| Super Sterol Ester (C10-30 Cholesterol/Lanosterol Esters) | 5.00 |
| Microcrystalline Wax | 2.00 |
| Ozokerite | 1.50 |
| Carnauba Wax | 1.25 |
| Candelilla Wax (refined) | 5.00 |

| **PART B** | |
|---|---|
| Castor Oil | 18.20 |
| C19-7711 Red 7 Lake (Sun Chemical Corp.) | 1.50 |
| C19-7712 Red 6 Lake (Sun Chemical Corp.) | 3.00 |
| C39-4433 Blue 1 Lake(Sun Chemical Corp.) | 0.95 |
| C33-8073 Cosmetic Yellow (Sun Chemical Corp.) | 1.00 |

| **PART C** | |
|---|---|
| Mica (Mearlmica MMCF) | 4.00 |
| Titanium Dioxide (and) Mica (and) Silica (Mearlmica MMCF) | 2.00 |
| Acorbyl Palmitate | 0.05 |

The lip stick disclosed in Table 9 may be formed by combining the ingredients of Part A while mixing and heating between a temperature of about 85°C to about 90°C, and until the mixture is clear. Separately, ingredients of Part B are mixed until particle size is less than about 25 microns. In some embodiments, a three-roll mill may be utilized to achieve the desired particle size. Then, Part B is added to Part A while continuing to mix until the mixture of Parts A and B is homogeneous. Then, the mixture of Parts A and B is cooled to about 75°C. After cooling, the ingredients of Part C are added while mixing and then the mixture is poured into molds at a temperature of about 70°C.

### Example 10: Hand Sanitizer

**Table 10**

| **Ingredients** | **Weight %** |
|---|---|
| **Part A** | |
| Alcohol (SDA-40) | 60.00 |
| Butylated hydroxytoluene (BHT) | 0.05 |
| Carbomer (Carbopol Ultrez 10 Polymer - Lubrizol) | 0.50 |
| PPG-3 Myristyl Methyl Ether | 0.25 |
| PPG-3 Isostearyl Methyl Ether | 1.25 |

| **PART B** | |
|---|---|
| Deionized Water | 34.95 |
| Glycerine | 2.00 |

| **PART C** | |
|---|---|
| Tromethamine (Tri Amino 30% solution) | 1.00 |

The hand sanitizer disclosed in Table 10 may be formed by dissolving the BHT in the alcohol, PPG-3 Myristyl Methyl Ether and PPG-3 Isostearyl Methyl Ether of Part A while mixing, and then adding in the carbomer while mixing with a propeller until it is all dissolved and uniform. Separately, Part B can be mixed together, and then Parts A and B can be combined and mixed until uniform. Part C is then added while mixing and the pH brought to between 5.5 to 7. Mixing is continued until uniform. The mixing can be switched to anchor mixing which can be continued to mix out any entrapped air if necessary.

### Example 11: Anti-Acne Facial Cream Cleanser

**Table 11**

| **Ingredients** | **Weight %** |
|---|---|
| **Part A** | |
| Deionised Water | 58.82 |
| Acrylates/C10-30 Alkyl Acrylate Crosspolymer | 0.60 |
| PRICERINE 9091 (Glycerin) | 3.00 |

| **Part B** | |
|---|---|
| Sodium Cocoyl Isethionate | 15.00 |

| **PART C** | |
|---|---|
| ARLASILK PTS (Stearamidopropyl PG-dimonium Chloride Phosphate and Cetyl Alcohol ) | 1.50 |
| CRODAFOS BES70 (Beheneth-30 Phosphate | 1.80 |
| (and) Cetyl Phosphate (and) Cetearyl Alcohol) | |
| CRODACOL S95 (Cetyl Alcohol) | 3.00 |
| PRISORINE 3515 (Isostearyl Alcohol) | 3.00 |
| PRIPURE 3759 (Squalane) | 1.50 |

| **Part D** | |
|---|---|
| Deionised Water | 1.00 |
| Sodium Hydroxide, 50% | 0.35 |

| **Part E** | |
|---|---|
| PPG-3 Isostearyl Methyl Ether | 5.00 |
| Benzoyl Peroxide, 75% Active | 3.33 |

| **Part F** | |
|---|---|
| Ethylhexylglycerin, Caprylyl Glycol | 0.10 |
| Polyethylene | 2.00 |

The anti-acne facial cream cleanser disclosed in Table 11 can be formed by combining the Part A ingredients and heating to 75°C while blade mixing for at least 10mins. Then, Part B can be added into Part A and the two Parts mixed until homogenous while maintaining the temperature. In a separate vessel, the Part C ingredients can be combined and mixed until homogenous while heating to 75°C. Under low to moderate homogenization, Part C may be added to Part A/B. The homogenization is continued for 1 minute. Part D is premixed and added to Part A/B/C under homogenization for 1 minute. The blade mixing is switched to sweep blade and the mixture is cooled. The Part E ingredients are weighed out at 1.5x more than stated in Table 11. In an ice bath under homogenization, the Part E ingredients are combined and homogenized at 25°-30°C until a smooth paste is formed. At 40°c, the main reaction vessel is placed in the ice bath. The actual weight of Part E is tared and added to the main vessel at 25°-30°C. Part F ingredients can then be added one at a time to the vessel with sweep mixing.

Example 12: Comparison of Viscosity between Alkoxylated Fatty Alcohols and Alkoxylated Fatty Alcohol Alkyl Ethers.

**Table 12**

| **Component** | **Viscosity(cSt)** |
|---|---|
| PPG-3 Isononanol Methyl Ether | 6.555 |
| PPG-3 Isononanoic Alcohol | 18.721 |
| | |
| PPG-3 Tridecyl Methyl Ether | 11.672 |
| PPG-3 Tridecyl Alcohol | 33.873 |
| | |
| PPG-3 Isostearyl Methyl Ether | 16.760 |
| PPG-3 Isostearyl Alcohol | 39.000 |

As shown in Table 12, akoxylated fatty alcohol and alkoxylated fatty alcohol alkyl ethers, which differ only in the end cap group can have different viscosities. For example, as shown in Table 5, alkoxylated fatty alcohol alkyl ethers can have lower viscosities than their alkoxylated fatty alcohol counterparts. A lower viscosity is desirable to improve spreadability in a personal care product which includes an alkoxylated fatty alcohol alkyl ether.
Kinematic viscosities were determined using a constant temperature bath and the appropriate capillary tube viscometers. The samples were loaded in to the capillary tubes and placed in the constant temperature bath and allowed to equilibrate. The viscosity measurements were that obtained using an equivalent method to the viscosity method as described in General Chapter <911> of the current USP compendia, where Chapter <911> is directed to capillary viscometer methods.

Example 13: Comparison of Viscosity in TiO₂ dispersions including Alkoxylated Fatty Alcohols and Alkoxylated Fatty Alcohol Alkyl Ethers.

**Table 13**

| **Component** | **Viscosity (cps)** |
|---|---|
| 40% TiO₂ + 60% PPG-3 Isostearyl Alcohol | 5,000 |
| 40% TiO₂ + 60% PPG-3 Isostearyl Methyl Ether | 3,500 |
| | |
| 50% TiO₂ + 50% PPG-3 Isostearyl Alcohol | 11,500 |
| 50% TiO₂ + 50% PPG-3 Isostearyl Methyl Ether | 12,500 |

An alkoxylated alcohol alkyl ether may improve viscosities in dispersions relative to a corresponding alkoxylated alcohol. In one embodiment, such as in a dispersion of TiO₂ and PPG-3 Isostearyl methyl ether, at TiO₂ amounts of greater than about 40% by weight, the alkoxylated alcohol alkyl ether may be less effective than the corresponding alkoxylated alcohol.

TiO₂ dispersions were made by dispersing the required amount into the test material. Then mixture is passed through a 3-roll mill a total of 3 times to ensure small particle size and a homogeneous smooth mixture. Viscosity of the mixture is then measured using a Brookfield viscometer.

Although the invention herein has been described with reference to particular embodiments, it is to be understood that these embodiments are merely illustrative of the principles and applications of the present invention.

## Claims

1. A compound for use in a personal care product having Formula X:
R₁O - A - R₂ (Formula X)
wherein:
i) A is (PO)ₙ, where P is a branched alkyl group having 3 carbons and n is an integer ranging from 2 to 10;
ii) R₂ is a methyl or ethyl group; and
iii) when R₂ is methyl, R₁ is a branched or unsaturated hydrocarbon having 16 to 24 carbons; or when R₂ is ethyl, R₁ is a branched or unsaturated hydrocarbon having 14 to 24 carbons.

2. The compound of claim 1, wherein when R₂ is methyl, n is 3 or n is 6.

3. The compound of claim 1, wherein when R₂ is ethyl, n ranges from 3 to 4.

4. The compound of claim 1, wherein n is 3.

5. The compound of claim 1, wherein R₁ is an isostearyl group.

6. A personal care product comprising:
(I) at least about 0.25 % by weight of a compound of Formula X:
R₁O - A - R₂ (Formula X)
wherein:
i) A is (PO)ₙ, where P is a branched alkyl group having 3 carbons and n ranges from 3 to 4;
ii) R₁ is a branched or unsaturated hydrocarbon having 8-24 carbons; and
iii) R₂ is a methyl group or an ethyl group; and
(II) a dermatologically acceptable excipient.

7. The personal care product of claim 6, wherein R₂ is a methyl group.

8. The personal care product of claim 6, wherein when R₂ is an ethyl group, R₁ is a branched or unsaturated hydrocarbon having 14-24 carbons.

9. A non-therapeutic method of applying a personal care product, the method comprising applying the personal care product of claim 6 to the human body.

10. A method of imparting conditioning to hair comprising applying the personal care product of claim 6 to hair.

11. A method of preserving the degree of hair color in synthetically colored hair comprising applying the personal care product of claim 6 to hair.

12. A method of imparting coloring to facial tissues comprising applying the personal care product of claim 6 to facial tissues.

## Patentansprüche

1. Verbindung zur Verwendung in einem Körperpflegeprodukt mit der Formel X:
R₁O-A-R₂ (Formel X),
wobei:
i) A für (PO)ₙ steht, wobei P für eine verzweigte Alkylgruppe mit 3 Kohlenstoffatomen steht und n für eine ganze Zahl im Bereich von 2 bis 10 steht;
ii) R₂ für eine Methyl- oder Ethylgruppe steht und
iii) dann, wenn R₂ für Methyl steht, R₁ für einen verzweigten oder ungesättigten Kohlenwasserstoff mit 16 bis 24 Kohlenstoffatomen steht; oder dann, wenn R₂ für Ethyl steht, R₁ für einen verzweigten oder ungesättigten Kohlenwasserstoff mit 14 bis 24 Kohlenstoffatomen steht.

2. Verbindung nach Anspruch 1, wobei dann, wenn R₂ für Methyl steht, n für 3 steht oder n für 6 steht.

3. Verbindung nach Anspruch 1, wobei dann, wenn R₂ für Ethyl steht, n im Bereich von 3 bis 4 liegt.

4. Verbindung nach Anspruch 1, wobei n für 3 steht.

5. Verbindung nach Anspruch 1, wobei R₁ für eine Isostearylgruppe steht.

6. Körperpflegeprodukt, umfassend:
(I) mindestens etwa 0,25 Gew.-% einer Verbindung der Formel X:
R₁O-A-R₂ (Formel X),
wobei:
i) A für (PO)ₙ steht, wobei P für eine verzweigte Alkylgruppe mit 3 Kohlenstoffatomen steht und n im Bereich von 3 bis 4 liegt;
ii) R₁ für einen verzweigten oder ungesättigten Kohlenwasserstoff mit 8-24 Kohlenstoffatomen steht und
iii) R₂ für eine Methyl- oder Ethylgruppe steht; und
(II) einen dermatologisch unbedenklichen Hilfsstoff.

7. Körperpflegeprodukt nach Anspruch 6, wobei R₂ für eine Methylgruppe steht.

8. Körperpflegeprodukt nach Anspruch 6, wobei dann, wenn R₂ für eine Ethylgruppe steht, R₁ für einen verzweigten oder ungesättigten Kohlenwasserstoff mit 14-24 Kohlenstoffatomen steht.

9. Nichttherapeutisches Verfahren zum Aufbringen eines Körperpflegeprodukts, bei dem man das Körperpflegeprodukt nach Anspruch 6 auf den menschlichen Körper aufbringt.

10. Verfahren zur Konditionierung von Haar, bei dem man das Körperpflegeprodukt nach Anspruch 6 auf Haar aufbringt.

11. Verfahren zur Konservierung des Haarfarbgrads in künstlich gefärbtem Haar, bei dem man das Körperpflegeprodukt nach Anspruch 6 auf Haar aufbringt.

12. Verfahren zum Versehen von Gesichtsgeweben mit Farbe, bei dem man das Körperpflegeprodukt nach Anspruch 6 auf Gesichtsgewebe aufbringt.

## Revendications

1. Composé pour une utilisation dans un produit de soin personnel possédant la formule X :
R₁O-A-R₂ (formule X)
i) A étant (PO)ₙ, où P est un groupe alkyle ramifié possédant 3 carbones et n est un entier dans la plage de 2 à 10 ;
ii) R₂ étant un groupe méthyle ou éthyle ; et
iii) lorsque R₂ est méthyle, R₁ est un hydrocarbure ramifié ou insaturé possédant 16 à 24 carbones ; ou lorsque R₂ est éthyle, R₁ est un hydrocarbure ramifié ou insaturé possédant 14 à 24 carbones.

2. Composé selon la revendication 1, où lorsque R₂ est méthyle, n est 3 ou n est 6.

3. Composé selon la revendication 1, où lorsque R₂ est éthyle, n est dans la plage de 3 à 4.

4. Composé selon la revendication 1, n étant 3.

5. Composé selon la revendication 1, R₁ étant un groupe isostéaryle.

6. Produit de soin personnel comprenant :
(I) au moins environ 0,25 % en poids d'un composé de formule X :
R₁O-A-R₂ (formule X)
i) A étant (PO)ₙ, où P est un groupe alkyle ramifié possédant 3 carbones et n est dans la plage de 3 à 4 ;
ii) R₁ étant un hydrocarbure ramifié ou insaturé possédant 8 à 24 carbones ; et
iii) R₂ étant un groupe méthyle ou un groupe éthyle ; et
(II) un excipient dermatologiquement acceptable.

7. Produit de soin personnel selon la revendication 6, R₂ étant un groupe méthyle.

8. Produit de soin personnel selon la revendication 6, R₂ étant un groupe éthyle, R₁ étant un hydrocarbure ramifié ou saturé possédant 14 à 24 carbones.

9. Procédé non thérapeutique d'application d'un produit de soin personnel, le procédé comprenant l'application du produit de soin personnel selon la revendication 6 au corps humain.

10. Procédé pour conférer un conditionnement à des cheveux comprenant l'application du produit de soin personnel selon la revendication 6 aux cheveux.

11. Procédé de préservation du degré de couleur capillaire dans des cheveux colorés synthétiquement comprenant l'application du produit de soin personnel selon la revendication 6 aux cheveux.

12. Procédé pour conférer une coloration à des tissus du visage comprenant l'application du produit de soin personnel selon la revendication 6 à des tissus du visage.
